# EUROPÄISCHE PATENTSCHRIFT

(11) **EP 1 671 709 B1**
(45) Veröffentlichungstag und Bekanntmachung des Hinweises auf die Patenterteilung: **02.05.2012**
(21) Anmeldenummer: 05027238.4
(22) Anmeldetag: 13.12.2005
(51) Int. Cl.: B08B 3/00, A61L 2/07, F22B 1/18

(54) **Verfahren und Vorrichtung zum Erzeugen von Heissdampf für Reinigungs- oder Dekontaminationszwecke**
Method and apparatus for the production of steam for cleaning and decontaminating purposes
Procédé et dispositif de production de vapeur pour le nettoyage et la décontamination

(30) Priorität: 13.12.2004 DE 102004062363
(43) Veröffentlichungstag der Anmeldung: 21.06.2006
(73) Patentinhaber: Kärcher Futuretech GmbH, 71409 Schwaikheim (DE)
(72) Erfinder: Kostron, Markus, 71686 Remseck (DE); Rentschler, Werner, Dr., 71332 Waiblingen (DE)
(74) Vertreter: Duhme, Torsten

(56) Entgegenhaltungen:
- CH-A- 204 601
- DE-C- 702 231
- US-A- 4 942 847
- US-A1- 2003 074 915

## Beschreibung

Die vorliegende Erfindung betrifft eine Vorrichtung zum Erzeugen von Heißdampf für Reinigungs- oder Dekontaminationszwecke, mit einem beheizbaren ersten Behälter zum Aufheizen einer Speiseflüssigkeit, die einen definierten Siedepunkt bei Normaldruck besitzt, und mit einem am Ausgang des ersten Behälters angeordneten Abscheider zum Extrahieren von Dampf, der beim Verdampfen von Speiseflüssigkeit entsteht.

Die Erfindung betrifft ferner ein Verfahren zum Erzeugen von Heißdampf für Reinigungs- oder Dekontaminationszwecke, mit den Schritten:
- Aufheizen einer Speiseflüssigkeit, die einen definierten Siedepunkt bei Normaldruck besitzt, in einem ersten Behälter,
- Zuführen der aufgeheizten Speiseflüssigkeit in einen Abscheider, und
- Extrahieren von Dampf, der beim Verdampfen von Speiseflüssigkeit entsteht, aus dem Abscheider.

Eine solche Vorrichtung und ein solches Verfahren sind aus DE 34 13 743 A1 und aus US 4 942 847 bekannt.

Dekontamination im Sinne der vorliegenden Erfindung ist das Entgiften oder Entseuchen von Personen oder Gegenständen, d.h. das Beseitigen und/oder Unwirksammachen von chemischen oder biologischen Kampfstoffen, mit denen Personen oder Gegenstände kontaminiert sind. Dementsprechend ist ein wesentlicher Anwendungsbereich der vorliegenden Erfindung die Entgiftung und/oder Entseuchung von militärischem Gerät. Die Erfindung ist hierauf jedoch nicht beschränkt und kann auch bei anderen Anwendungen eingesetzt werden, bei denen Heißdampf für Reinigungszwecke im weiteren Sinne benötigt wird. Die Erfindung kann daher auch im industriellen oder kommerziellen Bereich bei der Reinigung von Gegenständen und ebenso im medizinischen Bereich beim Desinfizieren oder Sterilisieren eingesetzt werden.

Die eingangs genannte DE 34 13 743 A1 beschreibt eine Vorrichtung zum Dekontaminieren von Gegenständen, insbesondere von Bekleidungsstücken. Dabei ist ein Dampferzeuger direkt hinter einer Brennkammer im Strömungsweg der aus dem Brenner austretenden Brenngase angeordnet. Der Dampferzeuger beinhaltet einen Dampfnacherhitzer im Umgebungsbereich der Brennkammer. Zwischen Dampfnacherhitzer und Brennkammer ist ein ringförmiger Wärmetauscher vorgesehen. Der Dampfnacherhitzer und der Luftwärmetauscher sind jeder für sich an einen gemeinsamen Auslassstutzen angeschlossen, an dem dann wahlweise Heißdampf, Heißluft oder ein Gemisch davon entnommen und einer nicht weiter dargestellten Dekontaminationskammer zugeführt werden kann. Die Erzeugung des Heißdampfes erfolgt in einem Rohrsystem, das von einen Wasserzulauf gespeist wird. Das Rohrsystem besitzt in Strömungsrichtung gesehen einen zunehmend größeren Querschnitt, um dem zunehmenden Volumenbedarf infolge der Verdampfung des Wassers Rechnung zu tragen. Am Ende mündet das Rohrsystem in einen als Kondensatraum bezeichneten Abscheider, aus dem etwa noch vorhandenes Heißwasser durch eine Kondensatleitung abgeleitet wird. Vom Kondensatraum gelangt der Dampf über den Dampfnacherhitzer zu dem Auslassstutzen.

Aus DE 1 127 365 ist eine weitere Vorrichtung zum Erzeugen von Heißdampf bekannt, wobei diese Vorrichtung nicht zu Dekontaminationszwecken, sondern zum Antreiben einer Turbine vorgesehen ist. Diese bekannte Vorrichtung besitzt einen ersten Behälter in Form einer ersten Rohrleitung, die in einen Abscheider mündet. Die erste Rohrleitung lässt sich über einen Brenner beheizen und der Abscheider dient dazu, Kondensat in den Speisekreislauf zurückzuführen. Der Dampf wird in einer zweiten Rohrleitung nacherhitzt (überhitzt) und dann der Turbine zugeführt. Ein besonderes Augenmerk wird bei dieser bekannten Vorrichtung daraufgelegt, dass die im Abscheider ausgeschiedenen Wassermengen nicht zu groß sind. Ferner sollen sprunghafte Druckänderungen vermieden werden. Weitere Vorrichtungen zur Dampferzeugung, allerdings nicht zu Dekontaminationszwecken, sind aus DE 1 065 858 B und DE 1 237 586 B bekannt.

Aus DE 38 35 857 A1 ist eine gattungsgemäße Vorrichtung bekannt, bei der ein Brenner am unteren Ende einer Brennkammer sitzt. Am oberen Ende der Brennkammer wird Speisewasser zugeführt, das an den Innenwänden der Brennkammer als Fallfilm nach unten läuft und dabei verdampft. Innerhalb der Brennkammer vermischen sich die Abgase des Brenners und der Wasserdampf, und das entstehende Gas-Dampf-Gemisch wird als Dekontaminationsmittel genutzt, wie dies verfahrenstechnisch aus DE 34 29 346 A1 bekannt ist. In einer alternativen Ausführung sitzt ein Brenner am Boden eines als Wasserbehälter ausgebildeten, nach oben geöffneten Rohrbündelsystems. Der aufsteigende Wasserdampf wird in einer über dem Rohrbündelsystem sitzenden Kammer überhitzt und anschließend wahlweise mit Frischluft und/oder Abgasen vermischt.

Aus DE 36 25 847 A1 ist ferner eine Dekontaminationskammer zum Dekontaminieren von Bekleidungs- und Ausrüstungsteilen bekannt. Die zu dekontaminierenden Gegenstände werden in der Kammer mit einem Gemisch aus Heißluft und Wasserdampf beaufschlagt. Die Erzeugung des Gemisches ist hier nicht weiter beschrieben.

Schließlich ist aus DE 1 454 671 ein Dampfstrahlreiniger bekannt, bei dem eine wässrige Lösung eines Reinigungsmittels in einer Heizwendel unter Druck erhitzt wird, sodass die Lösung teilweise verdampft, wenn sie in die freie Atmosphäre austritt.

Eine Trennung von Heißdampf und verbleibendem Flüssiganteil findet hier jedoch nicht statt.

Abgesehen von dem zuletzt genannten Dampfstrahlreiniger sind die bekannten Vorrichtungen und Verfahren alle dazu geeignet, Heißdampf oder ein Heißdampf-Gas-Gemisch zu erzeugen. Lediglich der zuletzt genannte Dampfstrahlreiniger stellt keinen separat nutzbaren Heißdampf zur Verfügung. Die bekannten Vorrichtungen und Verfahren haben sich jedoch als nicht effizient genug erwiesen, um 50 kg Trockendampf pro Stunde und mehr mit geringem Platzbedarf und Gewicht der Vorrichtung zu erzeugen. Darüber hinaus sind die bekannten Vorrichtungen und Verfahren einseitig auf die Erzeugung von Heißdampf und teilweise auf die Erzeugung eines Dampf-Gas-Gemisches ausgerichtet, das zum Dekontaminieren von Bekleidungsstücken in einer Dekontaminationskammer verwendet wird. Da bei einer Dekontamination häufig weitere Reinigungsaufgaben anfallen, wie beispielsweise die (Vor-)Reinigung von Großgerät, müssen die bekannten Vorrichtungen in der Praxis um weitere Anlagen ergänzt werden. Dies wirkt sich nachteilig auf die Mobilität und/oder Flexibilität der bekannten Vorrichtungen aus, was insbesondere für militärische Anwendungen oder auch bei Anwendungen im zivilen Katastrophenschutz von Nachteil ist.

Es ist daher eine Aufgabe der vorliegenden Erfindung, eine Vorrichtung der eingangs genannten Art sowie ein entsprechendes Verfahren anzugeben, mit denen eine bestimmte Menge Trockendampf pro Stunde auf effiziente Weise erzeugt werden kann. Darüber hinaus sollen die neue Vorrichtung und das neue Verfahren möglichst flexibel sein, d.h. sie sollen die Erzeugung von verschiedenen Dekontaminations- oder Reinigungsmitteln mit geringem Aufwand ermöglichen.

Diese Aufgabe wird gemäß einem Aspekt der vorliegenden Erfindung durch eine Vorrichtung der eingangs genannten Art gelöst, die einen Druckerzeuger aufweist, der dazu ausgebildet ist, die Speiseflüssigkeit in dem ersten Behälter unter einen höheren Druck als Normaldruck zu setzen, sowie ein Druckhalteelement, das zwischen dem Ausgang des Behälters und dem Abscheider angeordnet ist.

Gemäß einem weiteren Aspekt der Erfindung wird diese Aufgabe durch ein Verfahren der eingangs genannten Art gelöst, bei dem die Speiseflüssigkeit in dem ersten Behälter unter einem Druck aufgeheizt wird, der höher ist als der Normaldruck, und bei dem die Speiseflüssigkeit erst beim Zuführen in den Abscheider entspannt wird.

Die vorliegende Erfindung geht damit den Weg, die Speiseflüssigkeit beim Aufheizen unter einen (deutlich) höheren Druck als den Normaldruck (in aller Regel etwa gleich dem Umgebungsdruck) zu setzen. In einem bevorzugten Ausführungsbeispiel erfolgt das Aufheizen der Speiseflüssigkeit beispielsweise unter einem Druck von etwa 20 bar. Durch diesen hohen Druck lässt sich die Speiseflüssigkeit ohne Verdampfen auf eine Temperatur aufheizen, die deutlich höher liegt als die Siedetemperatur bei Normaldruck. Mit anderen Worten bleibt die aufgeheizte Speiseflüssigkeit aufgrund des höheren Drucks flüssig, obwohl ihre Temperatur weit jenseits der "normalen" Siedetemperatur liegt, bei der die Verdampfung einsetzt. In dem genannten Ausführungsbeispiel lässt sich beispielsweise Wasser als Speiseflüssigkeit auf eine Temperatur von bis zu 210°C aufheizen. Aufgrund des hohen Drucks findet jedoch (zunächst) keine Verdampfung statt.

Das Druckhalteelement der neuen Vorrichtung ist so ausgebildet, dass es den höheren Druck in dem ersten Behälter aufrechterhält, auch wenn Speiseflüssigkeit über das Ventil entnommen wird. Bei der Entnahme der aufgeheizten Speiseflüssigkeit über das Druckhalteelement wird die Speiseflüssigkeit dann abrupt entspannt, d.h. sie wird abrupt einem niedrigeren Druck ausgesetzt. Der niedrigere Druck kann der Normal- oder Umgebungsdruck sein oder auch ein höherer oder niedrigerer Druck, der jedoch in jedem Fall niedriger sein muss als der (höhere) Druck in dem ersten Behälter.

Durch das abrupte Absenken des auf die Speiseflüssigkeit einwirkenden Drucks verdampft ein Teil der Speiseflüssigkeit nahezu schlagartig. Der entstehende Dampfanteil und der verbleibende Flüssigkeitsanteil werden im Abscheider der neuen Vorrichtung voneinander getrennt. In einer bevorzugten Ausführung sitzt das Druckhalteelement dicht an dem Abscheider, sodass die Prozessschritte Verdampfen und Trennen bzw. Abscheiden praktisch in einem Schritt erfolgen.

Die neue Vorrichtung kann recht kleinbauend und mit einem im Vergleich zu bekannten Vorrichtungen relativ geringen Gewicht realisiert werden. Ein Grund hierfür liegt unter anderem darin, dass bei der neuen Vorrichtung auf eine separate Vorheizung der Speiseflüssigkeit verzichtet werden kann, weil mit dem im Abscheider abgeschiedenen Flüssigkeitsanteil stets eine hinreichende Menge vorgeheizter Speiseflüssigkeit zur Verfügung steht. Darüber hinaus arbeitet die neue Vorrichtung sehr effizient, da in dem ersten Behälter ein sehr guter Wärmeübergang stattfindet. Ein Grund hierfür liegt darin, dass der erste Behälter weitgehend oder sogar vollständig dampffrei gehalten ist, wodurch ein Filmsieden an den Innenwänden des Behälters und eine damit verbundene Degradation des Wärmeübergangs vermieden wird.

Darüber hinaus stellen die neue Vorrichtung und das neue Verfahren prozessbedingt schon zwei "Reinigungs- oder Dekontaminationsmittel" zur Verfügung, nämlich den Dampfanteil und den abgeschiedenen Flüssigkeitsanteil. Letzterer besetzt prozessbedingt eine Temperatur in der Größenordnung von etwa 100°C, wenn auf Normaldruck entspannt wird. Die neue Vorrichtung erzeugt somit Heißdampf und Heißwasser in einem gemeinsamen Prozessschritt, wenn Wasser als Speiseflüssigkeit verwendet wird. Damit entfällt die bei den bisher bekannten Dekontaminationsanlagen bestehende Notwendigkeit, zusätzlich nochmals Heißwasser zu erzeugen. Schließlich besitzen die neue Vorrichtung und das neue Verfahren den Vorteil, dass sich die Dampfmenge sehr einfach über die Druckdifferenz vor und nach dem Druckhalteventil, die Temperatur der Speiseflüssigkeit in dem ersten Behälter und/oder die Durchsatzänderung einstellen lässt.

Insgesamt stellt die vorliegende Erfindung damit eine sehr einfache, effiziente und trotzdem flexibel zu nutzende Alternative bereit, um Heißdampf (und gleichzeitig auch Heißwasser) zu erzeugen. Die genannte Aufgabe ist daher vollständig gelöst.

In einer Ausgestaltung der Erfindung weist der Abscheider einen Dampfaustritt auf, der in einen zweiten beheizbaren Behälter führt. Dementsprechend wird der erzeugte Heißdampf bei dem erfindungsgemäßen Verfahren in einem zweiten Behälter nacherhitzt.

Mithilfe dieser Ausgestaltung lässt sich auf einfache Weise überhitzter, trockener Dampf ohne Nassanteile erzeugen. Ein solcher Trockendampf ist für Dekontaminationszwecke besonders gut geeignet.

In einer weiteren Ausgestaltung besitzt die neue Vorrichtung einen Brenner, der eine Flamme und einen Abgasstrom erzeugt, wobei die Flamme den ersten Behälter beheizt und wobei der Abgasstrom den zweiten Behälter beheizt.

Diese Ausgestaltung ermöglicht eine besonders effiziente Erzeugung von Trockendampf, da die Energie des Brenners zweifach verwendet wird. Darüber hinaus ist das Aufheizen der Speiseflüssigkeit mithilfe eines Brenners eine besonders bevorzugte Variante für Vorrichtungen zur Dekontamination von militärischem Gerät oder zur Dekontamination in Katastrophenfällen, da Diesel- oder Vielstoffbrenner einen autarken Betrieb auch unter schwierigen Bedingungen ermöglichen. Alternativ hierzu kann die erfindungsgemäße Vorrichtung jedoch grundsätzlich auch mit anderen Heizquellen, beispielsweise einer elektrischen Heizquelle, betrieben werden.

In einer weiteren Ausgestaltung besitzt der Abscheider einen Flüssigkeitsaustritt, der mit einem Speisebehälter zum Bereitstellen der Speiseflüssigkeit verbunden ist.

Diese Ausgestaltung ermöglicht auf sehr einfache Weise einen äußerst effizienten Kreislaufbetrieb, in dem der abgeschiedene Flüssigkeitsanteil dem Prozess direkt wieder zugeführt werden kann. Da die Speiseflüssigkeit damit bereits auf eine hohe Temperatur vorgeheizt ist, arbeitet die neue Vorrichtung besonders effizient.

In einer weiteren Ausgestaltung beinhaltet die neue Vorrichtung einen Flüssigkeitsaustritt, über den abgeschiedene Speiseflüssigkeit wahlweise entnehmbar ist.

Bevorzugt ist dieser (ggf. weitere) Flüssigkeitsaustritt stromabwärts von dem Abscheider angeordnet, da der Abscheider auf diese Weise sehr einfach von Kalk- oder Schmutzablagerungen befreit werden kann. Darüber hinaus ermöglicht diese Ausgestaltung eine sehr einfache Entnahme von Heißwasser, was besonders bei der Dekontaminationen von militärischem Gerät oder in Katastrophenfällen von Vorteil ist.

In einer weiteren Ausgestaltung ist der Flüssigkeitsaustritt mit einem Druckhalteelement, insbesondere einem Siphon, versehen.

Ein Siphon ist eine sehr einfache und kostengünstige Möglichkeit, um einen definierten (niedrigen) Druck in dem Abscheider zu gewährleisten. Der damit einstellbare Druck ist jedoch begrenzt. Daher könnte der Flüssigkeitsaustritt alternativ hierzu auch mit einem zweiten Druckhalteventil versehen sein. Dies ermöglicht es beispielsweise, die abgeschiedene Speiseflüssigkeit unter einem Druck von etwa 2 bis 5 bar zu entnehmen. Besonders vorteilhaft ist die damit gegebene Flexibilität bei militärischen Einsätzen oder in Katastrophenfällen.

In einer weiteren Ausgestaltung weist der erste Behälter einen weiteren Ausgang auf, an dem aufgeheizte Speiseflüssigkeit unter dem höheren Druck entnehmbar ist. In einer besonders bevorzugten Ausgestaltung ist am Ausgang des ersten Behälters ein Dreiwegeventil angeordnet, von dem ein erster Ausgang über das Druckhalteventil mit dem Abscheider verbunden ist und von dem ein zweiter Ausgang zum Entnehmen der aufgeheizten Speiseflüssigkeit unter dem höheren Druck dient.

Diese Ausgestaltung ermöglicht auf sehr einfache und effiziente Weise einen weiteren "Reinigungsbetrieb", nämlich eine an sich bekannte Hochdruckreinigung mit einem Gemisch aus heißem Dampf und Wasser. Auch dies ist vor allem bei militärischen Einsätzen oder in Katastrophenfällen von großem Vorteil, weil häufig zusätzlich zu der Dekontamination von Bekleidung und persönlichen Ausrüstungsgegenständen auch Großgerät dekontaminiert werden muss. Die neue Vorrichtung dieser Ausgestaltung ermöglicht dies in einer sehr flexiblen und kompakten Realisierung.

In einer weiteren Ausgestaltung beinhaltet der Abscheider einen Zyklon (Fliehkraftabscheider), der einen nicht-verdampften Anteil der Speiseflüssigkeit und den Dampf voneinander trennt.

Die Verwendung eines Zyklons als Abscheider hat sich in einem Ausführungsbeispiel der neuen Vorrichtung als besonders effizient erwiesen. Der entstehende Dampfanteil kann nahezu vollständig abgetrennt und verwendet werden.

In einer weiteren Ausgestaltung der Erfindung beinhaltet die Vorrichtung eine Steuereinheit, die dazu ausgebildet ist, eine dem ersten Behälter zugeführte Menge an Speiseflüssigkeit umgekehrt proportional zur gewünschten Dampfmenge zu steuern. Vorzugsweise handelt es sich dabei um eine elektronische Steuereinheit und besonders bevorzugt um eine speicherprogrammierbare Steuerung (SPS).

Mithilfe einer solchen Steuereinheit können die Prozessparameter und insbesondere die erzeugte Menge an Heißdampf sehr einfach und effizient eingestellt werden. Die Zuführung einer größeren Menge an Speiseflüssigkeit hat zur Folge, dass sich die zugeführte Speiseflüssigkeit in dem ersten Behälter relativ gesehen weniger stark aufheizen kann (bei konstanter Heizleistung). Infolgedessen wird beim Entspannen der aufgeheizten Speiseflüssigkeit eine geringere Menge verdampft. Umgekehrt hat eine geringere Zuführung an Speiseflüssigkeit zur Folge, dass die zugeführte Speiseflüssigkeit stärker aufgeheizt wird und infolgedessen beim abrupten Entspannen mehr Dampf entsteht. Eine solche Steuerung der Dampfmenge ermöglicht es, die Speiseflüssigkeit in dem ersten Behälter mit konstanter Heizleistung zu beheizen. Dies ist besonders vorteilhaft, wenn zum Aufheizen der Speiseflüssigkeit ein Brenner verwendet wird, da eine Regelung der Heizleistung dann typischerweise nur im Taktbetrieb möglicht ist. Die bevorzugte Art der Steuerung ist demgegenüber sehr einfach, langlebig und robust.

In einer weiteren Ausgestaltung ist der Druckerzeuger eine volumetrisch fördernde Pumpe, deren Fördermenge vorzugsweise drehzahlabhängig ist.

Diese Ausgestaltung vereinfacht die zuvor beschriebene Steuerung der Dampfmenge noch weiter, da sich die Menge an zugeführter Speiseflüssigkeit mit einer derartigen Pumpe sehr einfach berechnen und variieren lässt. Darüber hinaus ist diese Art der Steuerung besonders robust und damit vor allem für militärische Anwendungen und Anwendungen im Katastrophenfall geeignet. Alternativ hierzu kann jedoch auch eine nicht volumetrisch fördernde Pumpe in Verbindung mit einem Durchflussmesser und einem Rückschlagventil nach der Pumpe eingesetzt werden.

In einer weiteren Ausgestaltung ist der Druckerzeuger dazu ausgebildet, die Speiseflüssigkeit in dem ersten Behälter unter einen Druck zwischen 10 und 30 bar zu setzen, vorzugsweise etwa 20 bar.

Diese Druckbereiche haben sich in praktischen Ausführungsbeispielen der Erfindung als besonders vorteilhaft erwiesen, um die benötigte Menge an Heißdampf in der beschriebenen Weise zu erzeugen. Die Verwendung von noch höheren Drücken ermöglicht eine weitere Steigerung der Dampfausbeute, stellt jedoch auch zunehmend höhere Anforderungen an die Belastbarkeit der verwendeten Vorrichtungsbestandteile, was im Hinblick auf die Robustheit und Mobilität der Vorrichtung von Nachteil ist.

In einer weiteren Ausgestaltung beinhaltet die Vorrichtung ferner eine Dekontaminationskammer mit einem Innenraum, in den der Heißdampf zuführbar ist, wobei die Dekontaminationskammer eine Heizung zum Aufheizen des Innenraums aufweist.

Die Zuführung von Heißdampf in eine Dekontaminationskammer ist an sich von den eingangs genannten Vorrichtungen und Verfahren bekannt. Die hier bevorzugte Ausgestaltung der Erfindung verwendet jedoch eine (zusätzliche) Heizung, mit der der Innenraum der Dekontaminationskammer zusätzlich zu der Zuführung des Heißdampfes aufgeheizt werden kann. Der Vorteil dieser Ausgestaltung liegt darin, dass die Kondensatbildung in der Dekontaminationskammer reduziert wird. Dadurch lässt sich die Dekontamination insgesamt zuverlässiger und effizienter durchführen.
In einer weiteren Ausgestaltung ist die Heizung im Bereich von Innenwänden der Dekontaminationskammer geführt. Vorzugsweise beinhaltet die Heizung zumindest einen Brenner, dessen Abgas mäanderförmig im Bereich der Innenwänden entlang geführt ist.

Diese Ausgestaltungen tragen zu einer weiteren Steigerung der Zuverlässigkeit und Effizienz der neuen Vorrichtung bei. Eine Beheizung der Innenwände der Dekontaminationskammer verhindert die Kondensatbildung besonders zuverlässig. Die Verwendung eines Brenners zur gesonderten Beheizung der Dekontaminationskammer ist darüber hinaus wiederum besonders für mobile Einsätze in Katastrophengebieten und bei militärischen Anwendungen geeignet.

Der Vollständigkeit halber sei darauf hingewiesen, dass die zuletzt genannten Ausgestaltungen auch für sich genommen als erfinderische Weiterbildungen bekannter Dekontaminationskammern betrachtet werden, d.h. die neuen Dekontaminationskammern können auch unabhängig von der hier vorgestellten Art der Heißdampferzeugung verwendet werden, um eine Dekontamination von Gegenständen effizienter und zuverlässiger durchzuführen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: eine schematische Darstellung eines Ausführungsbeispiels der neuen Vorrichtung zur Heißdampferzeugung,
- Fig. 2: eine schematische Darstellung einer Dekontaminationskammer, in die der Heißdampf zugeführt wird, der mit der in Fig. 1 gezeigten Vorrichtung erzeugt wurde, und
- Fig. 3: ein T-s-Diagramm zur Erläuterung des erfindungsgemäßen Verfahrens.

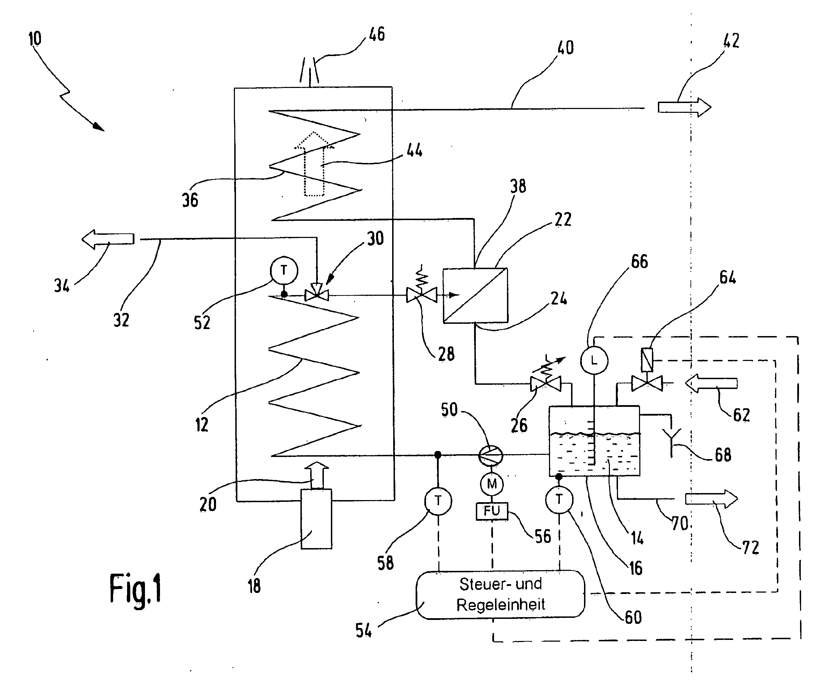

In Fig. 1 ist ein Ausführungsbeispiel der neuen Vorrichtung in seiner Gesamtheit mit der Bezugsziffer 10 bezeichnet.

Die Vorrichtung 10 beinhaltet einen ersten Behälter 12, der mit einer Speiseflüssigkeit 14 aus einem Vorratsbehälter 16 gespeist wird. Als Speiseflüssigkeit wird bevorzugt Wasser verwendet. Der Behälter 12 ist hier schematisch als Heizwendel dargestellt. Alternativ hierzu kann der Behälter 12 grundsätzlich eine beliebige Form besitzen, beispielsweise als Heizkessel ausgebildet sein.

Bei der Bezugsziffer 18 ist schematisch ein Brenner angedeutet, der eine Flamme 20 erzeugt. Bei dem Brenner 18 handelt es sich in einem bevorzugten Ausführungsbeispiel um einen Diesel- oder Vielstoffbrenner. Alternativ kann der erste Behälter 12 jedoch auch auf andere Weise, beispielsweise elektrisch beheizt werden.

Mit der Bezugsziffer 22 ist ein Abscheider bezeichnet, der zum Trennen von Dampf und Speiseflüssigkeit dient. In einem bevorzugten Ausführungsbeispiel handelt es sich hier um einen Zyklon (Fliehkraftabscheider). Ein solcher Zyklon besitzt einen beispielsweise zylinderförmigen Körper (hier nicht im Detail dargestellt), an dessen oberen Ende ein Einlass für die zu trennenden Medien vorgesehen ist. Die zu trennenden Medien (im Vorliegenden Dampf- und Flüssigkeitsanteil) werden etwa tangential zu dem Zylindermantel in den Abscheider eingespritzt. Da der Dampf leichter ist als der verbleibende Flüssigkeitsanteil, sammelt er sich im Wesentlichen im Inneren des Zylindermantels, während die Flüssigkeit aufgrund der Fliehkraft nach außen gegen den zylindermantel gedrückt wird und an diesem nach unten abläuft. Grundsätzlich kann die neue Vorrichtung jedoch auch mit einem anderen Abscheider zum Trennen von Dampf und Flüssigkeitsanteil betrieben werden.

Der Abscheider 22 besitzt an seinem unteren Ende einen Flüssigkeitsaustritt 24 zum Abschleusen des Flüssigkeitsanteils. Der Flüssigkeitsaustritt 24 ist über ein Druckhalteelement 26 mit dem Vorratsbehälter 16 verbunden. Das Druckhalteelement 26 ist in einem bevorzugten Ausführungsbeispiel ein Siphon. Alternativ hierzu kann es sich um ein einstellbares Druckhalteventil handeln, was insbesondere bevorzugt ist, wenn der Innenraum des Abscheiders 22 auf einem höheren Druck als dem Umgebungsdruck gehalten werden soll. Bevorzugt besitzt das Druckhalteventil 26 in diesem Fall einen Einstellbereich von etwa 1 bis 5 bar.

Mit der Bezugsziffer 28 ist ein weiteres Druckhalteventil bezeichnet, das am Eingang des Abscheiders 22 angeordnet ist. In einem bevorzugten Ausführungsbeispiel ist das Druckhalteventil 28 auf einen Haltedruck von zumindest 20 bar eingestellt. Der Abscheider 22 ist über das Druckhalteventil 28 mit einem ersten Ausgang des ersten Behälters 12 verbunden. Der erste Ausgang ist hier ein Ausgangszweig eines Dreiwegeventils 30. Ein zweiter Abzweig des Dreiwegeventils 30 bildet einen zweiten Ausgang 32. An dem Ausgang 32 kann bei Bedarf überhitzte Speiseflüssigkeit unter dem hohen Druck entnommen werden, der in dem ersten Behälter 12 vorherrscht. Die Entnahme von überhitzter Speiseflüssigkeit unter Hochdruck ist bei dem Pfeil 34 schematisch angedeutet. Praktisch handelt es sich hierbei um eine Betriebsart der Vorrichtung 10, die dem Betrieb eines herkömmlichen Hochdruckreinigers entspricht.

Oberhalb des ersten Behälters 12 ist hier ein zweiter Behälter 36 angeordnet, der hier vereinfacht ebenfalls als Heizwendel dargestellt ist. Der zweite Behälter 36 ist eingangsseitig mit einem Dampfaustritt 38 des Abscheiders 22 verbunden. Mit anderen Worten wird der zweite Behälter 36 mit Dampf gespeist, der aus dem Abscheider 22 entnommen wird. An einem Ausgang 40 des zweiten Behälters 36 kann der Dampf dann entnommen werden, was mithilfe des Pfeils 42 schematisch angedeutet ist. Bevorzugt wird der Dampf in dem zweiten Behälter 36 nacherhitzt (überhitzt) um Dampf ohne flüssigen Anteil in Form von Nebeltropfen zu erzeugen (Trockendampf). Zum Überhitzen des Dampfes in dem zweiten Behälter 36 wird in dem bevorzugten Ausführungsbeispiel der Abgasstrom 44 des Brenners 18 verwendet. In dem bevorzugten Ausführungsbeispiel ist der Abgasstrom 44 daher über bzw. durch die Heizwendel, die den zweiten Behälter 36 bildet, zu einem Abzug 46 geführt.

Die Vorrichtung 10 beinhaltet ferner einen Druckerzeuger 50, um die Speiseflüssigkeit in dem ersten Behälter 12 unter einen höheren Druck als Normaldruck zu setzen. In einem bevorzugten Ausführungsbeispiel handelt es sich bei dem Druckerzeuger um eine volumetrisch fördernde Pumpe, deren Fördermenge über die Drehzahl der Pumpe einstellbar ist. In einem Ausführungsbeispiel wird die Speiseflüssigkeit in dem ersten Behälter 12 mithilfe dieser Pumpe auf einen Druck von etwa 30 bar bis hin zu etwa 160 bar verdichtet. Gleichzeitig wird die Speiseflüssigkeit in dem ersten Behälter 12 mithilfe des Brenners 18 auf eine Temperatur von bis zu etwa 210°C aufgeheizt.

Bei der Bezugsziffer 52 ist ein Temperatursensor angedeutet, der am Ausgang des ersten Behälters 12 angeordnet ist. Mithilfe des Temperatursensors 52 kann die Temperatur der Speiseflüssigkeit in dem ersten Behälter 12 bestimmt werden. Wird die Vorrichtung 10 als Hochdruckreiniger betrieben, wird die aufgeheizte Speiseflüssigkeit vorzugsweise mit einer Temperatur von etwa 150°C und unter einem Druck von etwa 30-160 bar, vorzugsweise etwa 30-110 bar, an dem Ausgang 32 entnommen.

Bei der Bezugsziffer 54 ist vereinfacht eine Steuer- und Regeleinheit dargestellt (nachfolgend Steuereinheit). In einem bevorzugten Ausführungsbeispiel handelt es sich hier um eine elektronische speicherprogrammierbare Steuerung (SPS). Die Steuereinheit 54 stellt über einen Frequenzumrichter 56 die Drehzahl der Pumpe 50 ein. Mittelbar wird damit die Menge des erzeugten Trockendampfes eingestellt. Um eine optimale Einstellung aller Betriebsparameter zu ermöglichen, sind ferner zwei weitere Temperatursensoren 58, 60 vorgesehen. Der Temperatursensor 58 ist kurz hinter der Pumpe 50 (in Stromrichtung gesehen), also am Eingang des ersten Behälters 12 angeordnet. Mithilfe des Temperatursensors 58 lässt sich daher die Zulauftemperatur der Speiseflüssigkeit 14 beim Eintritt in den Behälter 12 bestimmen. Der Temperatursensor 60 sitzt demgegenüber im Bereich des Vorratsbehälters 16 um die Temperatur der Speiseflüssigkeit dort zu bestimmen. Die Steuereinheit 54 kann einen Kaltwasserzulauf 62 über ein Magnetventil 64 steuern und somit die Temperatur der Speiseflüssigkeit 14 in den Vorratsbehälter für einen optimalen Betrieb der Vorrichtung 10 einstellen.

Bei der Bezugsziffer 66 ist ferner ein Füllstandsmesser angedeutet, mit dessen Hilfe die Steuereinheit 54 den jeweiligen Füllstand der Speiseflüssigkeit 14 in den Vorratsbehälter bestimmen kann. Bei Bezugsziffer 68 ist ein Überlauf angedeutet, aus dem Speiseflüssigkeit bei einem zu hohen Füllstand im Behälter 16 austreten kann. Schließlich ist bei Bezugsziffer 70 ein Flüssigkeitsaustritt angedeutet, an dem hier Heißwasser unter Normaldruck entnommen werden kann (Bezugsziffer 72). Die Temperatur des Heißwassers 72 kann mithilfe der Steuereinheit 54, des Temperatursensors 60, des Kaltwasserzulaufs 62 und des aus dem Abscheider 22 rücklaufenden Speisewasseranteils auf einen gewünschten Temperaturwert eingeregelt werden.

Der Flüssigkeitsaustritt 70 dient in dem bevorzugten Ausführungsbeispiel auch zum Abschleusen von Speiseflüssigkeit zum Reinigen (Entkalten etc.) des Abscheiders 22, sofern dies erforderlich sein sollte.

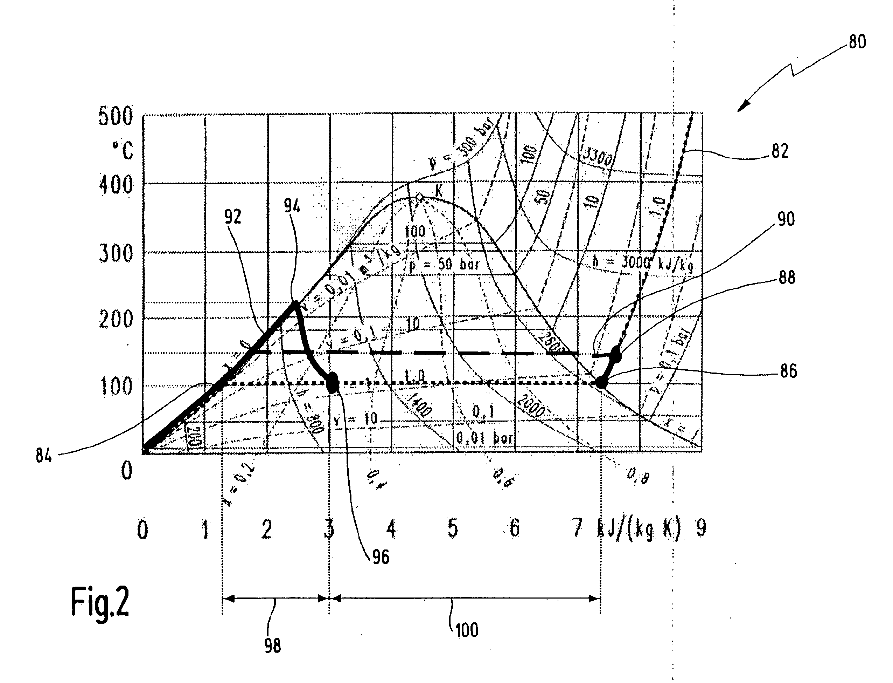

Die Funktionsweise der neuen Vorrichtung 10 (und dementsprechend ein Ausführungsbeispiel für das erfindungsgemäße Verfahren) lassen sich besonders gut anhand des Temperatur-Entropie-Diagramms 80 in Fig. 2 erläutern. In diesem Diagramm ist die Entropie auf der Abszisse des Diagramms aufgetragen. Entlang der Ordinate ist die Temperatur der Speiseflüssigkeit bzw. des entstehenden Dampfes aufgetragen. In durchgezogenen Linien verlaufen von links nach rechts die Isobaren. Gestrichelt sind in gleicher Richtung die Isochoren eingezeichnet. Schließlich geben die hyperbelartigen Verläufe die Kurven konstanter Enthalpie h (Isenthalpen) an.

In fetter punktierter Linie 82 ist der Verlauf eingezeichnet, wie er sich beim Verdampfen von Wasser unter Normaldruck ergibt, beispielsweise also in einem offenen Gefäß. Das Wasser wird ausgehend von 0°C zunächst auf den Siedepunkt 84 bei Normaldruck aufgeheizt, hier also etwa 100°C. Sobald dieser Siedepunkt erreicht ist, beginnt der Verdampfungsprozess. Dieser dauert solange an, bis sämtliches Wasser verdampft ist (Bezugsziffer 86). Die Temperatur und der Druck bleiben konstant bei etwa 100°C bzw. 1 bar (Normaldruck). Erst wenn alles Wasser verdampft ist (Punkt 86) lässt sich der entstandene Dampf weiter aufheizen, und zwar beispielsweise auf eine Temperatur von etwa 130°C (Punkt 88).

Bei der Bezugsziffer 90 ist demgegenüber in einer fetten, gestrichelten Linie ein Kurvenverlauf dargestellt, der sich ergibt, wenn Wasser unter einem etwas höheren Druck von beispielsweise bis zu 2 bar aufgeheizt und kontinuierlich verdampft wird. Aufgrund des höheren Drucks lässt sich das Wasser in diesem Fall bis auf etwa 130°C aufheizen. Anschließend bleibt die Temperatur weitgehend konstant oder kann nach vollständiger Verdampfung weiter erhöht werden.

Das neue Verfahren ist demgegenüber anhand des Kurvenverlaufs 92 (fette durchgezogene Linie) dargestellt, und zwar für den Fall, dass reines Wasser als Speiseflüssigkeit 14 verwendet wird. Lediglich der Vollständigkeit halber sei jedoch darauf hingewiesen, dass das neue Verfahren grundsätzlich auch mit anderen Speiseflüssigkeiten verwendet werden kann, beispielsweise wässrige Lösungen.

Nach dem neuen Verfahren wird die Speiseflüssigkeit zunächst auf eine Temperatur von etwa 210°C (Punkt 94), was nahezu ohne Verdampfung möglich ist, wenn die Speiseflüssigkeit in dem Behälter 12 unter einen entsprechend hohen Druck gesetzt wird.

In einem bevorzugten Ausführungsbeispiel beträgt der Druck in dem ersten Behälter 12 etwa 20 bar. Die überheizte Speiseflüssigkeit wird anschließend beim Durchgang durch das Druckhalteventil 28 abrupt entspannt. Diese Entspannung erfolgt isenthalp. In einem bevorzugten Ausführungsbeispiel erfolgt die Entspannung praktisch im selben Schritt, in dem die überheizte Speiseflüssigkeit in den Abscheider 22 eingespritzt wird. Durch die Entspannung sinkt der Druck auf den mithilfe des Druckhalteelements 26 eingestellten Wert (im gezeigten Beispiel etwa 1 bar) ab. Außerdem sinkt auch die Temperatur auf etwa 100°C ab. Gleichzeitig verdampft ein Teil der Speiseflüssigkeit, nämlich genau derjenige Anteil der bei einem kontinuierlichen Verdampfungsprozess gemäß Kurvenverlauf 82 beim Erreichen des Punktes 96 vorliegt. Bei den gewählten Prozessparametern verdampfen hier etwa 20 % der vorhandenen Speiseflüssigkeit. Etwa 80 % verbleiben in flüssiger Form und werden über den Flüssigkeitsaustritt 24 in den Vorratsbehälter 16 zurückgeführt. Der Dampfanteil ist in Fig. 2 bei der Bezugsziffer 98, der Flüssigkeitsanteil bei der Bezugsziffer 100 dargestellt.

Dadurch, dass der verbleibende Flüssigkeitsanteil 100 im Abscheider 22 von dem Dampfanteil 98 getrennt wird, "springt" der Kurvenverlauf 92 direkt zum Punkt 86, von wo aus die Überhitzung im zweiten Behälter 36 auf etwa 130°C erfolgt.

Die Menge des beim Eintritt in den Abscheider 22 entstehenden Dampfes lässt sich unter anderem über die Temperatur regeln, auf die die Speiseflüssigkeit in dem ersten Behälter 12 aufgeheizt wird. Wird bei einer kontinuierlichen Erzeugung von Trockendampf mehr Speiseflüssigkeit in den ersten Behälter eingespeist, verteilt sich die über den Brenner 18 zugeführte Energie auf eine größere Menge. Dementsprechend wird die Speiseflüssigkeit in dem Behälter 12 weniger stark erhitzt. Beim Durchtritt durch das Druckhalteventil 28 verdampft ein geringerer Anteil. Ein größerer Flüssigkeitsanteil wird dann in den Vorratsbehälter 16 zurückgeführt. Umgekehrt kann bei gleicher Brennerleistung eine höhere Temperatur der Speiseflüssigkeit erreicht werden, wenn weniger Speiseflüssigkeit in den Behälter 12 zugeführt wird. Aufgrund der geschilderten Zusammenhänge verdampft dann ein größerer Anteil und die Vorrichtung 10 erzeugt eine größere Menge an Trockendampf. Alternativ zu dieser bevorzugten Art der Einstellung der Dampfmenge könnte in anderen Ausführungsbeispielen der Erfindung jedoch auch die zugeführte Heizenergie und/oder der Druck in dem Behälter 12 variiert werden. Die bevorzugte Alternative ermöglicht jedoch eine sehr einfache Steuerung der Dampfmenge mithilfe der volumetrisch fördernden Pumpe 50. Andererseits können die verschiedenen Regelmechanismen auch miteinander kombiniert werden, was insbesondere dann vorteilhaft ist, wenn eine Flüssigkeitsentnahme bei 4 und/oder 72 zeitgleich zur Erzeugung von Trockendampf 42 erfolgt.

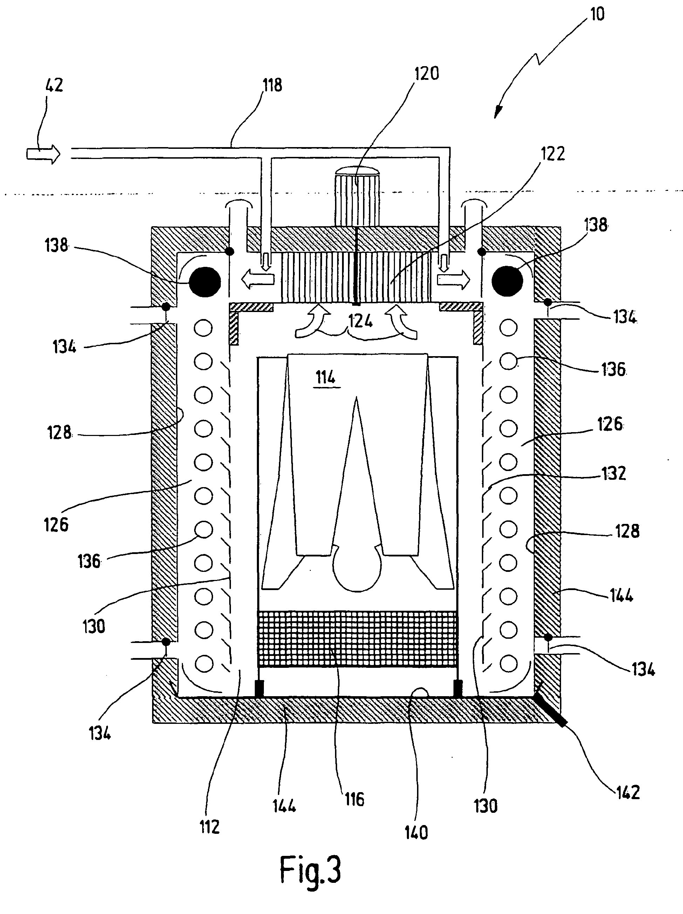

In einem besonders bevorzugten Ausführungsbeispiel, das vor allem für militärische Anwendungen und bei Katastropheneinsätzen von Vorteil ist, beinhaltet die Vorrichtung 10 ferner eine Dekontaminationskammer 110, wie sie in Fig. 3 schematisch dargestellt ist.

Die Dekontaminationskammer 110 besitzt einen Innenraum 112 zur Aufnahme von Gegenständen, die dekontaminiert werden sollen. In Fig. 3 ist beispielsweise ein sogenannter Overgarment 114 (ABC-Schutzanzug) dargestellt. Generell ist die Dekontaminationskammer 110 bevorzugt, wenn Bekleidungsteile oder andere allgemeine Ausrüstungsgegenstände von militärischen Verbänden dekontaminiert werden sollen, die höhere Temperaturen aushalten. Die Vorrichtung ist beispielsweise auch sehr gut zum Dekontaminieren von Versorgungs- und Packgefäßen geeignet. Generell kann die Kammer 110 dabei als Schleuse zwischen dem schmutzigen und dem reinen Bereich dienen.

Der Overgarment 114 ist hier an einem Beschickungswagen 116 aufgehängt. Er wird zum Dekontaminieren dem Trockendampf 42 ausgesetzt (ggf. auch einem Gemisch aus Trockendampf 42 und beigemischter Heißluft und/oder Brennerabgasen). Der Trockendampf 42 wird dazu über eine Leitung 118 in den Innenraum 112 der Dekontaminationskammer 110 zugeführt.

Bei der Bezugsziffer 120 ist ein Lüftermotor angedeutet, der einen Lüfter 122 antreibt. Vorzugsweise handelt es sich hierbei um einen Radiallüfter, der die Luft im Innenraum 112 der Kammer 110 umwälzt. Die Umwälzung erfolgt hier, indem der Radiallüfter 122 die Luft bzw. ein Dampf-Luft-Gasgemisch ansaugt (Pfeile 124) und anschließend in Heizkanäle 126 einbläst, die an den seitlichen Innenwänden 128 der Kammer 110 ausgebildet sind.

Die Heizkanäle 126 werden hier von Leitblechen 130 gebildet, die parallel zu den Innenwänden 128 der Kammer 110 angeordnet sind, und zwar in einem Abstand dazu, sodass ein lichter Zwischenraum verbleibt. Die Leitbleche 130 besitzen eine Vielzahl von Austrittsöffnungen 132, aus denen das umgewälzte Heißdampf-Luft-Gemisch austreten kann, um an dem zu dekontaminierenden Gegenstand (hier der Overgarment 114) entlang zu streichen.

Bei der Bezugsziffer 134 sind Eintrittsöffnungen mit Lüftungsklappen angedeutet, über die der Innenraum 112 der Kammer 110 mit Frischluft belüftet werden kann. Es gibt obere und untere Eintrittsöffnungen. Die durch die unteren Eintrittsöffnungen 134 einströmende Frischluft gelangt zunächst in die Heizkanäle 126 und wird dort, also erst in der Kammer 110, aufgeheizt. Dies ist ein Vorteil gegenüber den bislang bekannten Dekontaminationskammern, bei denen Luft bereits aufgeheizt in die Kammer zugeführt werden muss. Da die Mischung von Heißdampf und Luft erst in der Kammer 110 erfolgt, kann die Vorrichtung sehr kompakt realisiert werden. Über die oberen Eintrittsöffnungen 134 kann Frischluft zugeführt werden, die die Heizstrecke innerhalb der Kammer 110 nicht durchläuft. Die oberen Eintrittsöffnungen ermöglichen daher eine optimale Spülung der Kammer mit kalter Frischluft.

In den Heizkanälen 126 sind hier zwei mäanderförmige Abgasrohre 136 angeordnet. In den oberen Ecken der Vakuumkammer 10 (seitlich von dem Radiallüfter 122) befinden sich zwei Brenner 138. Mithilfe der Brenner kann der Innenraum 112 der Kammer 110 insgesamt beheizt werden. Zusätzlich dienen die Abgasrohre 136 der Brenner 138 zum Aufheizen der über die Lüftungsklappen 134 zugeführten Luft sowie zum (weiteren) Aufheizen des in dem Innenraum 112 umgewälzten Luft-Dampf-Gemisches.

Mit der Bezugsziffer 140 ist eine am Boden der Kammer 110 angeordnete Auffangwanne bezeichnet, die einen Ablauf 142 besitzt, über den Kondenswasser aus der Kammer 110 abgeführt werden kann. Des Weiteren ist bei der Bezugsziffer 144 eine Isolierung angedeutet, die den Innenraum 112 nach außen hin thermisch isoliert.

Die Verwendung von zwei Brennern 138 zum Beheizen des Innenraums 112 ist hier bevorzugt, um eine möglichst gleichmäßige Beheizung des Innenraums und der ggf. über die Lüftungsklappen 134 zugeführten Frischluft zu erreichen. Alternativ hierzu könnte der Innenraum 112 jedoch auch mit nur einem Brenner oder auf andere Weise beheizt werden. Maßgeblich für die bevorzugte Dekontaminationskammer 110 ist dabei in erster Linie, dass eine Kondensatbildung an den Innenwänden 128 der Kammer 110 verhindert wird. Daher ist es von besonderem Vorteil, die Heizkanäle 126 in der dargestellten Form an den Innenwänden 128 entlang zu führen.

Die dargestellte Kammer 110 erzeugt einen sehr effizienten Strom aus Heißdampf, Frischluft und Gas, der alle in der Kammer befindlichen Gegenstände sehr gleichmäßig durchströmt. Die Menge aller drei Stoffe ist unabhängig voneinander einstellbar. Von großem Vorteil ist dabei, dass das aufgeheizte Gemisch die zu dekontaminierenden Gegenstände von unten anströmt, weil die natürliche Konvektion den gewünschten Strom unterstützt. Insbesondere kann bei der bevorzugten Kammer auf strömungsunterstützende Abstandhalter zwischen einzelnen Kleidungsstücken verzichtet werden. Die Verwendung von Beschickungswagen ermöglicht eine schnelle und gefahrlose Entnahme von dekontaminierten Gegenständen und die Abstände zwischen den Gegenständen sind mit der Anordnung auf dem Beschickungswagen definiert. Außerdem erfolgt die Beladung der Kammer stets weitgehend gleich, so dass der Luft- und Gasstrom in der Kammer stets weitgehend gleich ist.

## Patentansprüche

1. Vorrichtung zum Erzeugen von Heißdampf (42) für Reinigungs- oder Dekontaminationszwecke, mit einem beheizbaren ersten Behälter (12) zum Aufheizen einer Speiseflüssigkeit (14), die einen definierten Siedepunkt (84) bei Normaldruck besitzt, und mit einem am Ausgang (30) des ersten Behälters (12) angeordneten Abscheider (22) zum Extrahieren von Dampf, der beim Verdampfen von Speiseflüssigkeit (14) entsteht, **gekennzeichnet durch** einen Druckerzeuger (50), der dazu ausgebildet ist, die Speiseflüssigkeit (14) in dem ersten Behälter (12) unter einen höheren Druck (94) als Normaldruck zu setzen, und **durch** ein Druckhalteelement (28), das zwischen dem Ausgang (30) des Behälters (12) und dem Abscheider (22) angeordnet ist.

2. Vorrichtung nach Anspruch 1, **dadurch gekennzeichnet, dass** der Abscheider (22) einen Dampfaustritt (38) aufweist, der in einen zweiten beheizbaren Behälter (36) führt.

3. Vorrichtung nach Anspruch 2, **gekennzeichnet durch** einen Brenner (18), der eine Flamme (20) und einen Abgasstrom (44) erzeugt, wobei die Flamme (20) den ersten Behälter (12) beheizt und wobei der Abgasstrom (44) den zweiten Behälter (36) beheizt.

4. Vorrichtung nach einem der Ansprüche 1 bis 3, **dadurch gekennzeichnet, dass** der Abscheider (22) einen Flüssigkeitsaustritt (24) besitzt, der mit einem Speisebehälter (16) zum Bereitstellen der Speiseflüssigkeit (14) verbunden ist.

5. Vorrichtung nach einem der Ansprüche 1 bis 4, **gekennzeichnet durch** einen Flüssigkeitsaustritt (70), über den abgeschiedene Speiseflüssigkeit (14) wahlweise entnehmbar ist.

6. Vorrichtung nach Anspruch 4 oder 5, **dadurch gekennzeichnet, dass** der Flüssigkeitsaustritt (24, 70) mit einem Druckhalteelement insbesondere einem Siphon, versehen ist.

7. Vorrichtung nach einem der Ansprüche 1 bis 6, **dadurch gekennzeichnet, dass** der erste Behälters (12) einen weiteren Ausgang (32) aufweist, an dem aufgeheizte Speiseflüssigkeit (14) unter dem höheren Druck entnehmbar ist.

8. Vorrichtung nach Anspruch 7, **dadurch gekennzeichnet, dass** am Ausgang des ersten Behälters (12) ein Dreiwegeventil (30) angeordnet ist, von dem ein erster Ausgang über das Druckhalteventil mit dem Abscheider (22) verbunden ist und von dem ein zweiter Ausgang (32) zum Entnehmen der aufgeheizten Speiseflüssigkeit (34) unter dem höheren Druck dient.

9. Vorrichtung nach einem der Ansprüche 1 bis 8, **dadurch gekennzeichnet, dass** der Abscheider (22) einen Zyklon beinhaltet, der einen nicht-verdampften Anteil der Speiseflüssigkeit (100) und den Dampf (98) voneinander trennt.

10. Vorrichtung nach einem der Ansprüche 1 bis 9, **gekennzeichnet durch** eine Steuereinheit (54), die dazu ausgebildet ist, eine dem ersten Behälter (12) zugeführte Menge an Speiseflüssigkeit (14) umgekehrt proportional zur gewünschten Dampfmenge (98) zu steuern.

11. Vorrichtung nach einem der Ansprüche 1 bis 10, **dadurch gekennzeichnet, dass** der Druckerzeuger (50) eine volumetrisch fördernde Pumpe ist, deren Fördermenge vorzugsweise drehzahlabhängig ist.

12. Vorrichtung nach einem der Ansprüche 1 bis 11, **dadurch gekennzeichnet, dass** der Druckerzeuger (50) dazu ausgebildet ist, die Speiseflüssigkeit (14) in dem ersten Behälter (12) unter einen Druck von mehr als 10 bar, vorzugsweise etwa 20 bar, zu setzen.

13. Vorrichtung nach einem der Ansprüche 1 bis 12, ferner **gekennzeichnet durch** eine Dekontaminationskammer (110) mit einem Innenraum (112), in den der Heißdampf (42) zuführbar ist, wobei die Dekontaminationskammer (110) eine Heizung (126, 138) zum Aufheizen des Innenraums (112) aufweist.

14. Vorrichtung nach Anspruch 13, **dadurch gekennzeichnet, dass** die Heizung (126, 138) im Bereich von Innenwänden (128) der Dekontaminationskammer (110) geführt wird.

15. Verfahren zum Erzeugen von Heißdampf (42) für Reinigungs- oder Dekontaminationszwecke, mit den Schritten:
- Aufheizen einer Speiseflüssigkeit (14), die einen definierten Siedepunkt (94) bei Normaldruck besitzt, in einem ersten Behälter (12),
- Zuführen der aufgeheizten Speiseflüssigkeit (14) in einen Abscheider (22), und
- Extrahieren von Dampf (98), der beim Verdampfen von Speiseflüssigkeit (12) entsteht, aus dem Abscheider (22),
**dadurch gekennzeichnet,**
- **dass** die Speiseflüssigkeit (14) in dem ersten Behälter (12) unter einem Druck (94) aufgeheizt wird, der höher ist als der Normaldruck, und
- **dass** die Speiseflüssigkeit (14) erst beim Zuführen in den Abscheider (22) entspannt wird.

## Claims

1. Apparatus for the production of hot steam (42) for cleaning or decontaminating purposes, comprising a heatable first vessel (12) for heating a feed liquid (14) which has a defined boiling point (84) at normal pressure, and comprising a separator (22), arranged at the outlet (30) of the first vessel (12), for the extraction of steam which occurs during the evaporation of the feed liquid (14), **characterized by** a pressure generator (50) which is designed for putting the feed liquid (14) in the first vessel (12) under a pressure (94) higher than normal pressure, and by a pressure-holding element (28) which is arranged between the outlet (30) of the vessel (12) and the separator (22).

2. Apparatus according to Claim 1, **characterized in that** the separator (22) has a steam outlet (38) which leads into a second heatable vessel (36).

3. Apparatus according to Claim 2, **characterized by** a burner (18) which generates a flame (20) and an exhaust gas stream (44), the flame (20) heating the first vessel (12) and the exhaust gas stream (44) heating the second vessel (36).

4. Apparatus according to one of Claims 1 to 3, **characterized in that** the separator (22) has a liquid outlet (24) which is connected to a feed vessel (16) for providing the feed liquid (14).

5. Apparatus according to one of Claims 1 to 4, **characterized by** a liquid outlet (70), via which separated feed liquid (14) can be selectively extracted.

6. Apparatus according to Claim 4 or 5, **characterized in that** the liquid outlet (24, 70) is provided with a pressure-holding element (26), in particular a siphon.

7. Apparatus according to one of Claims 1 to 6, **characterized in that** the first vessel (12) has a further outlet (32) at which heated feed liquid (14) under the higher pressure can be extracted.

8. Apparatus according to Claim 7, **characterized in that**, at the outlet of the first vessel (12), a three-way valve (30) is arranged, of which a first outlet is connected to the separator (22) via the pressure-holding valve and of which a second outlet (32) serves for extracting the heated feed liquid (34) under the higher pressure.

9. Apparatus according to one of Claims 1 to 8, **characterized in that** the separator (22) comprises a cyclone which separates a non-evaporated fraction of the feed liquid (100) and the steam (98) from one another.

10. Apparatus according to one of Claims 1 to 9, **characterized by** a control unit (54) which is designed for controlling a quantity of feed liquid (14) supplied to the first vessel (12) in inverse proportion to the desired steam quantity (98).

11. Apparatus according to one of Claims 1 to 10, **characterized in that** the pressure generator (50) is a volumetric-delivery pump, the delivery rate of which is preferably dependent on rotational speed.

12. Apparatus according to one of Claims 1 to 11, **characterized in that** the pressure generator (50) is designed for putting the feed liquid (14) in the first vessel (12) under a pressure of more than 10 bar, preferably of about 20 bar.

13. Apparatus according to one of Claims 1 to 12, furthermore **characterized by** a decontamination chamber (110) with an inner space (112) into which the hot steam (42) can be supplied, the decontamination chamber (110) having a heater (126, 138) for heating the inner space (112).

14. Apparatus according to Claim 13, **characterized in that** the heater (126, 138) is arranged in the region of inner walls (128) of the decontamination chamber (110).

15. Method for the production of hot steam (42) for cleaning or decontaminating purposes, comprising the steps of:
- heating of a feed liquid (14) which has a defined boiling point (94) at normal pressure in a first vessel (12),
- supplying the heated feed liquid (14) into a separator (22), and
- extracting steam (98) which occurs during the evaporation of feed liquid (12) from the separator (22),
**characterized**
- **in that** the feed liquid (14) in the first vessel (12) is heated under a pressure (94) which is higher than the normal pressure, and
- **in that** the feed liquid (14) is expanded only when being supplied into the separator (22).

## Revendications

1. Dispositif de production de vapeur d'eau surchauffée (42) dans un but de nettoyage ou de décontamination, le dispositif présentant
un premier récipient (12) apte à être chauffé et servant à chauffer un liquide d'alimentation (14) dont le point d'ébullition (84) sous pression normale est défini
et un séparateur (22) disposé à la sortie (30) du premier récipient (12) et permettant d'extraire la vapeur d'eau surchauffée formée par vaporisation du liquide d'alimentation (14),
**caractérisé par**
un dispositif (50) d'établissement de pression configuré pour placer le liquide d'alimentation (14) présent dans le premier récipient (12) sous une pression (94) plus élevée que la pression normale et
par un élément (28) de maintien de la pression disposé entre la sortie (30) du récipient (12) et le séparateur (22).

2. Dispositif selon la revendication 1, **caractérisé en ce que** le séparateur (22) présente une sortie (38) de vapeur qui conduit dans un deuxième récipient (36) apte à être chauffé.

3. Dispositif selon la revendication 2, **caractérisé par** un brûleur (18) qui produit une flamme (20) et un écoulement (44) de gaz de combustion, la flamme (20) chauffant le premier récipient (12) et l'écoulement (44) de gaz de combustion chauffant le deuxième récipient (36).

4. Dispositif selon l'une des revendications 1 à 3, **caractérisé en ce que** le séparateur (22) possède une sortie (24) de liquide qui est reliée à un récipient d'alimentation (16) qui délivre le liquide d'alimentation (14).

5. Dispositif selon l'une des revendications 1 à 4, **caractérisé par** une sortie (70) de liquide par laquelle le liquide d'alimentation (14) séparé peut être soutiré sélectivement.

6. Dispositif selon les revendications 4 ou 5, **caractérisé en ce que** la sortie (24, 70) de liquide est dotée d'un élément (26) de maintien de pression et en particulier d'un siphon.

7. Dispositif selon l'une des revendications 1 à 6, **caractérisé en ce que** le premier récipient (12) présente une autre sortie (32) par laquelle du liquide d'alimentation (14) à pression plus élevée et chauffé peut être soutiré.

8. Dispositif selon la revendication 7, **caractérisé en ce qu'**à la sortie du premier récipient (12) est disposée une soupape (30) à trois voies dont une première sortie est reliée au séparateur (22) par l'intermédiaire de la soupape de maintien de pression et dont une deuxième sortie (32) sert à soutirer le liquide d'alimentation (34) chauffé et sous pression plus élevée.

9. Dispositif selon l'une des revendications 1 à 8, **caractérisé en ce que** le séparateur (22) contient un cyclone qui sépare l'une de l'autre la partie non vaporisée du liquide d'alimentation (100) et la vapeur d'eau (98).

10. Dispositif selon l'une des revendications 1 à 9, **caractérisé par** une unité de commande (54) configurée pour commander la quantité de liquide d'alimentation (14) apportée au premier récipient (12) en proportion inverse à la quantité souhaitée de vapeur d'eau (98).

11. Dispositif selon l'une des revendications 1 à 10, **caractérisé en ce que** le dispositif (50) d'établissement de pression est une pompe volumétrique dont le débit dépend de préférence de la vitesse de rotation.

12. Dispositif selon l'une des revendications 1 à 11, **caractérisé en ce que** le dispositif (50) d'établissement de pression est configuré pour placer le liquide d'alimentation (14) présent dans le premier récipient (12) sous une pression supérieure à 10 bars et de préférence d'environ 20 bars.

13. Dispositif selon l'une des revendications 1 à 12, **caractérisé en outre par** une chambre de décontamination (110) dotée d'un espace intérieur (112) dans lequel la vapeur d'eau surchauffée (42) peut être amenée, la chambre de décontamination (110) présentant un chauffage (126, 138) qui permet de chauffer l'espace intérieur (112).

14. Dispositif selon la revendication 13, **caractérisé en ce que** le chauffage (126, 138) est conduit au niveau des parois intérieures (128) de la chambre de décontamination (110).

15. Procédé de production de vapeur d'eau surchauffée (42) dans un but de nettoyage ou de décontamination, le procédé comportant les étapes qui consistent à :
- chauffer dans un premier récipient (12) un liquide d'alimentation (14) dont le point d'ébullition (94) sous pression normale est défini,
- amener le liquide d'alimentation (14) chauffé dans un séparateur (22) et
- extraire du séparateur (22) la vapeur d'eau (98) qui se forme lors de la vaporisation du liquide d'alimentation (12),
**caractérisé en ce que**
le liquide d'alimentation (14) présent dans le deuxième récipient (12) est chauffé à une pression (94) supérieure à la pression normale et
**en ce que** le liquide d'alimentation (14) n'est détendu qu'après avoir été amené dans le séparateur (22).
